# EUROPEAN PATENT SPECIFICATION

(11) **EP 2 748 511 B1**
(45) Date of publication and mention of the grant of the patent: **14.10.2015**
(21) Application number: 12826543.6
(22) Date of filing: 17.12.2012
(51) Int. Cl.: F16M 13/02, A61M 16/00

(54) **PORTABLE CONNECTOR FOR SECURING PORTABLE MEDICAL DEVICES**
TRAGBARER VERBINDER ZUR SICHERUNG TRAGBARER MEDIZINISCHER VORRICHTUNGEN
RACCORD PORTABLE POUR FIXER DES DISPOSITIFS MÉDICAUX PORTABLES

(30) Priority: 23.12.2011 US 201161579706 P
(43) Date of publication of application: 02.07.2014
(73) Proprietor: Koninklijke Philips N.V., 5656 AE Eindhoven (NL)
(72) Inventor: SEIVER, Adam Jacob, 5656 AE Eindhoven (NL)
(74) Representative: Steffen, Thomas
(86) International application number: PCT/IB2012/057361
(87) International publication number: WO 2013/093749

(56) References cited:
- WO-A1-03/040608
- DE-A1-102005 009 315
- DE-U1-202010 008 112
- US-A1- 2006 231 705

## Description

### FIELD OF THE INVENTION

The invention relates to vacuum connectors, particularly for vacuum connectors for medical devices.

### BACKGROUND OF THE INVENTION

Patients managed with mechanical ventilators frequently require transport among multiple diagnostic and treatment areas of the hospital. During transport it is physiologically desirable for the patient and convenient for the clinical staff to be able to continue mechanical ventilation uninterrupted. Some ventilators for assisted respiration are portable enough to be easily transported with the patient by hanging the hook-shaped ventilator handle on the bed headboard or bedrails.

A holding implement is known from WO 03/040608A1. This holding implement discloses all the technical features of the preamble of claims 1,5, and 6 and comprises a tray connected to a suction element via a parallelogram guide. The tray is both cushioned and dampened by a pneumatic spring, which acts upon the parallelogram guide, whereby reducing the loads of the suction element while also protecting the device from impact loads.

Once at the location, with transfer of the patient from the bed onto operating room table, or transfer to catheterization lab or CT gantry, finding a place to hang the ventilator that is near the patient may be challenging. Even though the ventilator is portable enough to be attached to the bed, once the patient is moved onto e.g. the CT gantry the bed would ordinarily be removed so that there are no remaining places to put the ventilator.

Accordingly, it is a problem to find connection facilities for portable medical devices in a process where a patient is transported between different locations and different medical devices.

Accordingly, the inventor of the present invention has appreciated that improvements for enabling better attachment possibilities for portable medical devices would be of benefit, and has in consequence devised the present invention.

### SUMMARY OF THE INVENTION

It would be advantageous to achieve improved possibilities for finding attachment options for portable medical devices. It would also be desirable to enable portable medical devices to be attached to various surfaces such as surfaces of larger medical equipment such as CT scanners or the like. In particular, it may be seen as an object of the present invention to provide a connector that solves the above mentioned problems of finding attachment facilities of portable medical devices, or other problems, of the prior art.

To better address one or more of these concerns, in a first aspect of the invention a portable connector device for providing a secure connection structure for a portable medical device is presented that comprises
- the connection structure shaped to enable connection with a connection feature of the portable medical device,
- one or more vacuum connectors for making a vacuum connection with a surface, where the vacuum connector has a cavity circumscribed by a rim for making contact with the surface, where the one or more vacuum connectors are connected with the connection structure,
- a vacuum provider for providing a vacuum in the cavity,
- an air pressure detector for measuring the air pressure in the cavity of the vacuum connector, where the air pressure detector is configured to provide data related to the measured air pressure to an associated external monitoring device capable of generating an alarm in dependence of the provided data.

By means of the vacuum connectors the portable connector device may be attached to various surfaces such as surfaces of medical equipment used e.g. for examination of patients. Accordingly, as the patient is moved from a bed to a medical examination device the portable connection device may be vacuum connected to a surface of the medical examination device and the portable medical device may be attached to the connection structure of the portable connection device.

Clearly it is extremely important that portable medical device is securely attached so that the patient does not risk that the portable medical device falls down. Accordingly, the vacuum connection must not fail. The possibility of monitoring the air pressure and generating an alarm in response to detection of a decreasing air pressure reduces the risk of a vacuum failure.

Thus, if a continuous increase in air pressure is detected or if the air pressure increases above a given threshold hospital personnel may be called in automatically by the external monitoring device to take care of the critical situation.

In an embodiment the monitoring device may be comprised by the portable medical device. Since portable medical devices may have monitoring and alarm functions associated with the primary medical functions of the device, e.g. ventilation functions, these monitoring and alarm functions may advantageously be utilized also for monitoring the measured air pressure provided by the vacuum connectors and for generating an alarm if a too high air pressure is detected.

In an embodiment the vacuum provider may be an air connector for enabling connection with an external vacuum generator. In order to improve the reliability of the vacuum connectors, the vacuum may be generated by an external vacuum generator such as an external vacuum pump which is connected to the portable connection device via the air connector. Advantageously, the external vacuum generator may continuously provide suction to the vacuum connectors so that the risk of vacuum failures may be reduced, particularly for long term attachments of portable medical devices.

In an embodiment the vacuum generator is comprised by the portable medical device. Since portable medical devices such as ventilators may have vacuum functions associated with the primary medical functions of the device, e.g. ventilation functions, this vacuum function may advantageously be utilized also for providing a vacuum for the cavity of the vacuum connectors.

A second aspect of the invention relates to a portable medical device comprising
- one or more vacuum connectors for making a vacuum connection with a surface, where the vacuum connector has a cavity circumscribed by a rim for making contact with the surface,
- a vacuum provider for providing a vacuum in the cavity,
- an air pressure detector for measuring the air pressure in the cavity of the vacuum connector, where the air pressure detector is configured to provide data related to the measured air pressure,
- an alarm device capable of generating an alarm in dependence of the measured air pressure.

This second aspect of the invention may provide one or more of the advantages of the first aspect since the second aspect mainly differ from the first aspect by integrating the components of the portable connector device, except the connector structure, with the portable medical device. Advantageously, the vacuum connectors may be embedded in the portable medical device so that they can be hidden when they are not in use.

A third aspect of the invention relates to a method for providing a secure connection structure for a portable medical device, the method comprises
- attaching a portable connector device according to the first aspect to a surface,
- attaching the portable medical device to the connection structure of the portable connector device,
- monitoring the air pressure in the cavity of the portable connector device,
- supplying the data related to the measured air pressure to the associated external monitoring device.

In summary the invention relates to a portable vacuum connector device. The connector device can be attached to various surfaces such as surfaces of a CT scanner or other medical equipment by means of vacuum cups or connectors. The vacuum connector device has a connection structure such as a rail to which a portable medical device can be attached via its connection feature, e.g. a hook. Accordingly, the connector device makes may make it easier to let the portable medical device follow the patient as the patient is moved from location to location. An air pressure sensor is provided for monitoring the vacuum level and for enabling generation of an alarm via an external monitoring device such as the portable medical device.

In general the various aspects of the invention may be combined and coupled in any way possible within the scope of the invention. These and other aspects, features and/or advantages of the invention will be apparent from and elucidated with reference to the embodiments described hereinafter.

### BRIEF DESCRIPTION OF THE DRAWINGS

Embodiments of the invention will be described, by way of example only, with reference to the drawings, in which
Fig. 1 shows a portable connector device 100,
Fig. 2 shows a cross sectional view of a vacuum connector 102 of the portable connector device,
Fig. 3A shows connection of a portable medical device 201 with the portable connector device 100,
Fig. 3B shows a portable medical device 300 having integrated vacuum connectors 102.

### DESCRIPTION OF EMBODIMENTS

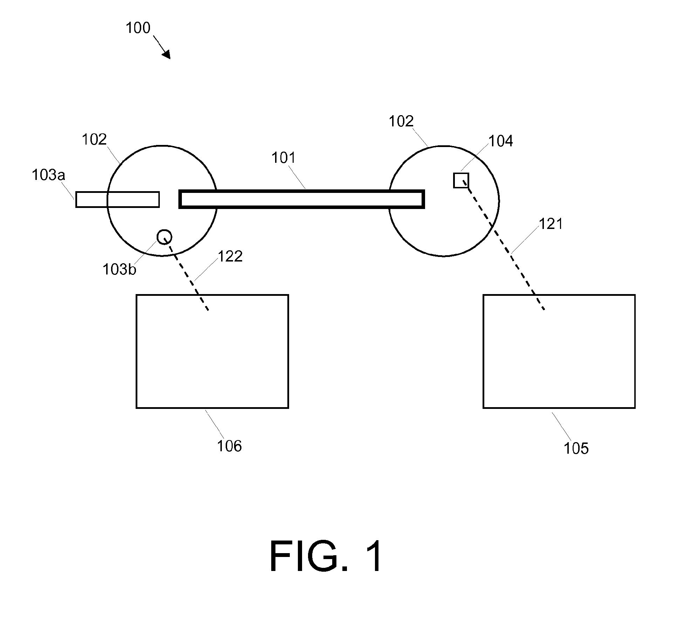

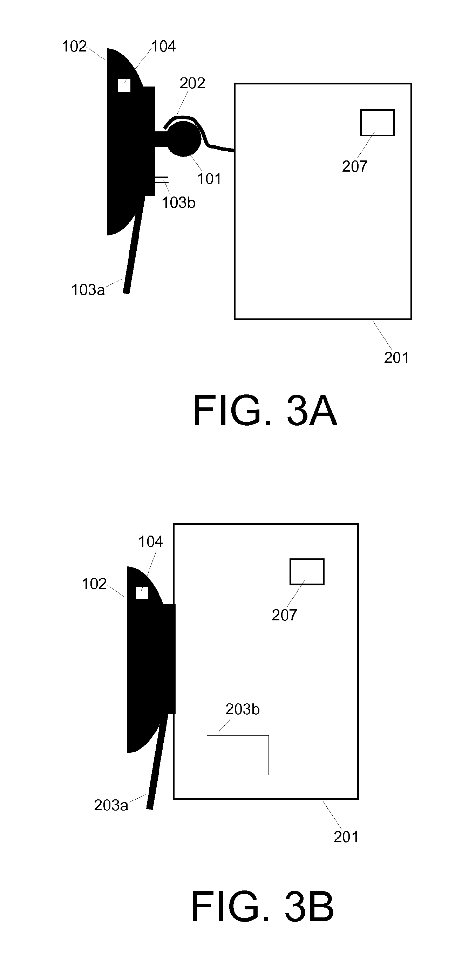

Fig. 1 shows a portable connector device 100 for providing secure connection possibilities for a portable medical device such as a medical ventilator. Some portable medical devices have a hook or similar connection feature 202 (see Fig. 3A) so that they can be hung on the rail of a bed when the patient is moved. However, when the patient is moved from the bed, e.g. onto a CT gantry in a CT scanner room, there may not be any convenient places to place or hang the portable medical device. The portable connector device 100 is designed to provide a connection possibility for such portable medical devices. For example, the connector device may be attached to the surface of a CT scanner and the portable medical device can be connected to a rail of the connector device 100.

The connector device 100 is shown with two vacuum connectors 102, but may also be configured with only one, alternatively with more than two vacuum connectors such as three, four or more vacuum connectors.

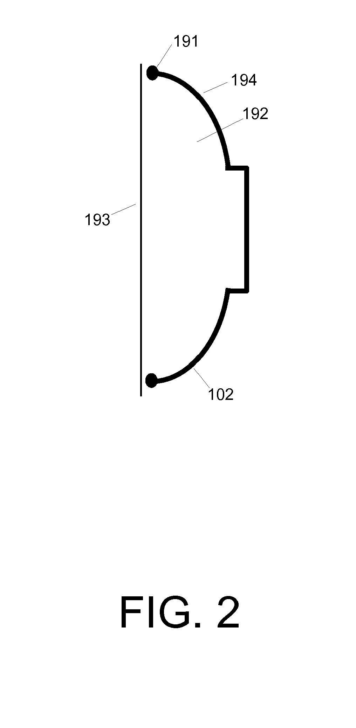

Fig. 2 shows a cross sectional view of the vacuum connector 102. The vacuum connector comprises a shell 194 and a rim 191which circumscribes the opening of the shell. The shell and the rim together define a cavity 192. The rim may be made of elastic material such as rubber for making an airtight connection with a surface 193. When the air pressure in the cavity 192 is reduced relative to the surrounding air pressure so as to generate a vacuum in the cavity the rim 191 is pressed against the surface 193 so that the vacuum connector is connected to the surface 193 and, thereby, makes a vacuum connection with the surface 193, such as a suitable surface of a CT scanner or other medical apparatus. The shell 194 may be made of solid or elastic material and may be integrally formed with the rim 191. The general configuration of vacuum connector 102 as described here is known. The general vacuum connector 102 may be known as vacuum or suction cups.

The vacuum in the cavity of the vacuum connectors is provided by a vacuum provider. The vacuum provider may be embodied by a lever 103a which acts on a mechanism which creates a vacuum by increasing a volume of the cavity 192. Similarly, the vacuum provider may be embodied by a pump (not shown) integrated with the vacuum connector 102 and which is capable of creating a vacuum in the cavity 192. Alternatively, the vacuum provider may be embodied by an air connector 103b which is connectable with an external vacuum generator 106 such as an electric vacuum pump for creation of a vacuum, e.g. via an air hose 122. The air connector 103b may comprise a valve for ensuring that the vacuum is not lost in case of loss of the vacuum from the external vacuum generator 106. It is understood that the creation of a vacuum in the cavity 192 requires an air tight connection of the rim 191 with a surface 193.

In an embodiment the vacuum generator 106 is part of the portable medical device 201. For example, a medical ventilator has a built-in pump system for ventilating patients. This built-in pump system may advantageously be utilized also as the vacuum generator 106 for creation of a vacuum for the vacuum connectors 102.

The portable connector device 100 further comprises a connection structure 101, such as a rail, ring, a hook or other suitable connection structure shaped to enable connection with a the portable medical device, possibly via a connection feature 202 of the portable medical device. The connection structure 101 is connected with the vacuum connectors 102. For example, a rail 101 may connect two connection structures as shown in Fig. 1.

The portable connector device 100 further comprises an electrical air pressure detector 104 such as a piezoresistive or capacitive air pressure detector for measuring the air pressure in the cavity 192 of the vacuum cup. The electrical output measurement signal from the air pressure detector 104 may be processed by an electrical circuit comprised by the connector device 100 into data values related to the measured air pressure. The unprocessed measurement signal or the processed measurement signal is made available or supplied to an associated external monitoring device 105 capable of generating an alarm in dependence of the provided data. The signal may be made available or supplied via an electrical connector of the portable connector device through a wired or wire-less connection 121.

The external monitoring device 105 may be configured to only monitor the measured vacuum data from the air pressure detector 104, or the monitoring device 105 may be comprised by the portable medical device 201. Portable medical devices 201 such as medical ventilators may have an input for monitoring various measured signals and, therefore, such an input may be used for monitoring the measured air pressure data or equivalent processed data related to the measured air pressure. If the data format of the measured air pressure signal is not compatible with the input of the portable medical device, the measured air pressure signal may be converted into a signal, i.e. a data signal which is related to the measured air pressure, which is compatible with the input of the portable medical device. For example, the data signal which is related to the measured air pressure may be a simple binary signal which is low (e.g. a digital zero) when the air pressure is low enough to provide a secure connection of the vacuum connectors 102, and which is high (e.g. a digital one) when the air pressure becomes too high so that the vacuum connection is at risk. Alternatively, the measured air pressure signal may be processed to form a voltage signal having maximum and minimum voltages corresponding to the maximum and minimum voltages which can be sensed by the input of the portable medical device 201. The conversion of the measured air pressure to the binary signal may be performed by the electrical circuit (not shown) comprised by the connector 100.

In an embodiment the portable connector device 100 is configured to be collapsible to enable easy storage and transport of the device. The collapsible design may be realized by making the connection structure bendable by connecting parts of the connection structure with one or more hinges 131.

The portable connector device may be configured in various ways. For example, the connection structure 101 may be a rope provided with vacuum connectors 102 at both ends; the connection structure 101 may be a ring connected to one or more vacuum connectors 102; or the connection structure 101 may be a hook attached to a single vacuum connector 102.

The geometry and dimensions of the portable connector device may be specifically designed to enable attachment to specific medical equipment. For example, a CT scanner may only have a few places and limited areas where the connector device 100 can be attached and, therefore, the geometry and dimensions of the connector 100 may be specifically designed to meet the mounting possibilities of the medical equipment. Also the vacuum connectors 102 and especially the surface properties of the rims 191, such as the softness or elasticity of the surface of the rims 191 may be selected so at to obtain the optimum connection properties with e.g. non-smooth surfaces 193.

Fig. 3A shows an exemplary embodiment of a portable connector device 100 which is mechanically connectable via the connection structure 101 to portable medical device 201 via the hook 202 of the medical device 201. The air pressure sensor 104 is connectable with the portable medical device 201 (e.g. via a wired connection 121) so that an alarm device 207 comprised by the medical device 201 can generate an alarm in case of critical vacuum level in the cavity 192. The alarm device 207 may be a general alarm device configured to generate alarms in dependence of other measured medical conditions, or specifically configured for generating alarms in dependence of the measured air pressure.

Fig. 3B shows a portable medical device 201 having integrated vacuum connectors 102 for making a vacuum connection with an external surface, where the suction cups are integrated with the portable medical device 201. The vacuum connectors are equivalent to vacuum connectors of Fig. 1 and may have an integrated vacuum provider in the form of a manual pump or lever 203a for establishing a vacuum in the cavity 192 as in the portable connector device 100 in Fig. 1. Alternatively, a vacuum pump 203b of the portable medical device 201 may be connected to the vacuum connector 102 for making a vacuum in the cavity 192. An air pressure detector is provided similarly to the other embodiments for measuring the air pressure in the cavity of the vacuum connector. The air pressure detector is connected to an alarm device 207 which is equivalent to the alarm device 207 of Fig. 3A.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single electronic circuit such as a processor or other unit may fulfill the functions of several items recited in the claims. The mere fact that certain measures are recited in mutually different dependent claims does not indicate that a combination of these measures cannot be used to advantage. Any reference signs in the claims should not be construed as limiting the scope.

## Claims

1. A portable connector arrangement including:
a portable connector device (100) for providing a secure connection structure (101) for a portable medical device (201), the connector device comprises
- the connection structure (101) shaped to enable connection with a connection feature (202) of the portable medical device,
- one or more vacuum connectors (102) for making a vacuum connection with a surface, where the vacuum connector has a cavity (192) circumscribed by a rim (191) for making contact with the surface, where the one or more vacuum connectors are connected with the connection structure,
- a vacuum provider (103 a, 103b) for providing a vacuum in the cavity,
- an air pressure detector (104) for measuring the air pressure in the cavity of the vacuum connector, where the air pressure detector (104) is configured to provide data related to the measured air pressure; and being **characterized by** further comprising: an external monitoring device (105) arranged to receive the provided data and to generate an alarm in dependence of the provided data.

2. A portable connector arrangement according to claim 1, where the monitoring device (105) is comprised by the portable medical device (201).

3. A portable connector arrangement according to claim 1, where the vacuum provider is an air connector (103b) for enabling connection with an external vacuum generator (106).

4. A portable connector arrangement according to claim 3, where the external vacuum generator (106) is comprised by the portable medical device (201).

5. A portable medical device (300) comprising
- one or more vacuum connectors (102) for making a vacuum connection with a surface, where the vacuum connector has a cavity circumscribed by a rim for making contact with the surface,
- a vacuum provider (203a, 203b) for providing a vacuum in the cavity,
- an air pressure detector (104) for measuring the air pressure in the cavity of the vacuum connector, where the air pressure detector (104) is configured to provide data related to the measured air pressure,
- an alarm device (207) external to said one or more vacuum connectors (102), said alarm device being capable of generating an alarm in dependence of the measured air pressure.

6. A method for providing a secure connection structure for a portable medical device (201), the method comprises
- attaching a portable connector device (100) according to claim 1 to a surface (193),
- attaching the portable medical device (201) to the connection structure (101) of the portable connector device (100),
- monitoring the air pressure in the cavity (192) of the portable connector device (100), the method being **characterized by**:
- supplying the measured air pressure or data related to the measured air pressure to an associated external monitoring device (105); and
- generating an alarm with the external monitoring device (105) in dependence of the provided data.

## Patentansprüche

1. Tragbare Verbinderanordnung, die Folgendes umfasst:
eine tragbare Verbindervorrichtung (100) zum Bereitstellen einer sicheren Verbindungsstruktur (101) für eine tragbare medizinische Vorrichtung (201), wobei die Verbindervorrichtung Folgendes umfasst:
- die Verbindungsstruktur (101), die geformt ist, um eine Verbindung mit einem Verbindungsmerkmal (202) der tragbaren medizinischen Vorrichtung zu ermöglichen,
- einen oder mehrere Unterdruckverbinder (102) zum Herstellen einer Unterdruckverbindung mit einer Oberfläche, wobei der Unterdruckverbinder einen Hohlraum (192) hat, der von einer Einfassung (191) umgeben ist, um Kontakt mit der Oberfläche herzustellen, wobei der eine oder mehrere Unterdruckverbinder mit der Verbindungsstruktur verbunden sind,
- eine Unterdruckbereitstellungseinheit (103a, 103b) zum Bereitstellen eines Unterdrucks in dem Hohlraum,
- einen Luftdruckdetektor (104) zum Messen des Luftdrucks in dem Hohlraum des Unterdruckverbinders, wobei der Luftdruckdetektor (104) konfiguriert ist, um Daten in Bezug auf den gemessenen Luftdruck bereitzustellen; und **dadurch gekennzeichnet, dass** sie weiterhin Folgendes umfasst:
eine externe Überwachungsvorrichtung (105), die dafür vorgesehen ist, die bereitgestellten Daten zu empfangen und in Abhängigkeit von den bereitgestellten Daten einen Alarm zu erzeugen.

2. Tragbare Verbinderanordnung nach Anspruch 1, wobei die Überwachungsvorrichtung (105) in der tragbaren medizinischen Vorrichtung (201) enthalten ist.

3. Tragbare Verbinderanordnung nach Anspruch 1, wobei die Unterdruckbereitstellungseinheit ein Luftverbinder (103b) zum Ermöglichen einer Verbindung mit einem externen Unterdruckgenerator (106) ist.

4. Tragbare Verbinderanordnung nach Anspruch 3, wobei der externe Vakuumgenerator (106) in der tragbaren medizinischen Vorrichtung (201) enthalten ist.

5. Tragbare medizinische Vorrichtung (300), die Folgendes umfasst:
- einen oder mehrere Unterdruckverbinder (102) zum Herstellen einer Unterdruckverbindung mit einer Oberfläche, wobei der Unterdruckverbinder einen Hohlraum hat, der von einer Einfassung umgeben ist, um Kontakt mit der Oberfläche herzustellen,
- eine Unterdruckbereitstellungseinheit (203a, 203b) zum Bereitstellen eines Unterdrucks in dem Hohlraum,
- einen Luftdruckdetektor (104) zum Messen des Luftdrucks in dem Hohlraum des Unterdruckverbinders, wobei der Luftdruckdetektor (104) konfiguriert ist, um Daten in Bezug auf den gemessenen Luftdruck bereitzustellen,
- eine Alarmvorrichtung (207) außerhalb des genannten einen oder mehrerer Unterdruckverbinder (102), wobei die genannte Alarmvorrichtung in der Lage ist, in Abhängigkeit von dem gemessenen Luftdruck einen Alarm zu erzeugen.

6. Verfahren zum Bereitstellen einer sicheren Verbindungsstruktur für eine tragbare medizinische Vorrichtung (201), wobei das Verfahren Folgendes umfasst:
- Anbringen einer tragbaren Verbindervorrichtung (100) nach Anspruch 1 an einer Oberfläche (193),
- Anbringen der tragbaren medizinischen Vorrichtung (201) an der Verbindungsstruktur (101) der tragbaren Verbindungsvorrichtung (100),
- Überwachen des Luftdrucks in dem Hohlraum (192) der tragbaren Verbindervorrichtung (100), wobei das Verfahren **gekennzeichnet ist durch**:
- Zuführen des gemessenen Luftdrucks oder von Daten bezüglich des gemessenen Luftdrucks zu einer zugehörigen externen Überwachungsvorrichtung (105); und
- Erzeugen eines Alarms mit der externen Überwachungsvorrichtung (105) in Abhängigkeit von den bereitgestellten Daten.

## Revendications

1. Ensemble connecteur portable comprenant :
un dispositif connecteur portable (100) pour fournir une structure de connexion sécurisée (101) pour un dispositif médical portable (201), le dispositif connecteur comprenant
- la structure de connexion (101) profilée pour permettre une connexion avec un élément de connexion (202) du dispositif médical portable,
- un ou plusieurs connecteurs à vide (102) pour réaliser une connexion de vide avec une surface, le connecteur à vide possédant une cavité (192) entourée par un rebord (191) pour faire un contact avec la surface, le ou les connecteurs à vide étant connectés à la structure de connexion,
- un fournisseur de vide (103 a, 103b) pour fournir un vide dans la cavité,
- un détecteur de pression d'air (104) pour mesurer la pression de l'air dans la cavité du connecteur à vide, le détecteur de pression d'air (104) étant conçu pour fournir des données se rapportant à la pression d'air mesurée ; et étant **caractérisé en ce qu'**il comprend en outre : un dispositif de surveillance externe (105) agencé pour recevoir les données fournies et pour générer une alarme en fonction des données fournies.

2. Ensemble connecteur portable selon la revendication 1, où le dispositif de surveillance (105) est compris par le dispositif médical portable (201).

3. Ensemble connecteur portable selon la revendication 1, où le fournisseur de vide est un connecteur d'air (103b) pour permettre la connexion avec un générateur de vide externe (106).

4. Ensemble connecteur portable selon la revendication 3, où le générateur de vide externe (106) est compris par le dispositif médical portable (201).

5. Dispositif médical portable (300) comprenant
- un ou plusieurs connecteurs à vide (102) pour réaliser une connexion de vide avec a surface, où le connecteur à vide possède une cavité entourée par un rebord pour faire un contact avec la surface,
- un fournisseur de vide (203a, 203b) pour fournir un vide dans la cavité,
- un détecteur de pression d'air (104) pour mesurer la pression de l'air dans la cavité du connecteur à vide, le détecteur de pression d'air (104) étant conçu pour fournir des données se rapportant à la pression d'air mesurée,
- un dispositif d'alarme (207) externe audit ou auxdits connecteur(s) à vide (102), ledit dispositif d'alarme étant capable de générer une alarme en fonction de la pression d'air mesurée.

6. Procédé pour fournir une structure de connexion sécurisée pour un dispositif médical portable (201), le procédé comprenant
- la fixation d'un dispositif connecteur portable (100) selon la revendication 1 sur une surface (193),
- la fixation du dispositif médical portable (201) à la structure de connexion (101) du dispositif connecteur portable (100),
- la surveillance de la pression d'air dans la cavité (192) du dispositif connecteur portable (100), le procédé étant **caractérisé par** :
- la fourniture de la pression d'air mesurée ou de données se rapportant à la pression d'air mesurée à un dispositif de surveillance externe associé (105) ; et
- la génération d'une alarme avec le dispositif de surveillance externe (105) en fonction des données fournies.
